# EUROPEAN PATENT SPECIFICATION

(11) **EP 2 035 421 B1**
(45) Date of publication and mention of the grant of the patent: **02.07.2014**
(21) Application number: 07718845.6
(22) Date of filing: 04.05.2007
(51) Int. Cl.: C07D 487/04, A61K 51/04, A61K 31/505, A61K 31/519, A61P 25/28

(54) **2-Arylpyrazolo[l,5-alpha]pyrimidin-3-yl acetamide derivatives as ligands for translocator protein (18 kDa)**
2-Arylpyrazolo[l,5-alpha]pyrimidin-3-yl-acetamid-Derivate als Liganden für das Translokatorprotein (18 kDa)
Dérivés de l'acétamide de 2-arylpyrazolo[l,5-alpha]pyrimidin-3-yle comme ligands pour une protéine de translocation (18 kDa)

(30) Priority: 19.05.2006 AU 2006902715
(43) Date of publication of application: 18.03.2009
(73) Proprietor: THE UNIVERSITY OF SYDNEY, Sydney, New South Wales 2006 (AU)
(72) Inventor: KASSIOU, Michael, Bexley, Nsw 2207 (AU); JAMES, Michelle, Louise, Caringbah, Nsw 2229 (AU)
(74) Representative: Grund, Martin
(86) International application number: PCT/AU2007/000598
(87) International publication number: WO 2007/134362

(56) References cited:
- US-A- 5 114 944
- C. THOMINIAUX ET AL: "Improved synthesis of the peripheral benzodiazepine receptor ligand [<11>C]DPA-713 using [<11>C]methyl triflate" APPLIED RADIATION AND ISOTOPES, ELSEVIER, OXFORD, GB, vol. 64, no. 5, 1 May 2006 (2006-05-01), pages 570-573, XP005331228 ISSN: 0969-8043
- S. SELLERI ET AL.: "A Novel Selective GABA A alpha 1 Receptor Agonist Displaying Sedative and Anxiolytic-like Properties in Rodents" JOURNAL OF MEDICINAL CHEMISTRY, vol. 48, no. 21, 2005, pages 6756-6760, XP002574166 ISSN: 0022-2623
- ZHANG M-R ET AL: "Development of a New Radioligand, N-(5-fluoro-2-phenoxyphenyl)-N-(2-Ä 18FÜfluoroethyl-5-methoxybenzyl)acetamide, for PET Imaging of Peripheral Benzodiazepine Receptor in Primate Brain" JOURNAL OF MEDICINAL CHEMISTRY, AMERICAN CHEMICAL SOCIETY, WASHINGTON, US, vol. 47, no. 9, 22 April 2004 (2004-04-22) , pages 2228-2235, XP002997542 ISSN: 0022-2623
- SELLERI S. ET AL.: '2-Arylpyrazolo[1,5-a]pyrimidin-3-yl Acetamides. New Potent and Selective Peripheral Benzodiazepine Receptor Ligands' BIOORG. MED. CHEM. vol. 9, no. 10, 2001, pages 2661 - 2671, XP008090470
- SELLERI S. ET AL.: 'Insight into 2-phenylpyrazolo[1,5-a]pyrimidin-3-yl acetamides as peripheral benzodiazepine receptor ligands: Synthesis, biological evaluation and 3D-QSAR investigation' BIOORG. MED. CHEM. vol. 13, no. 16, 2005, pages 4821 - 4834, XP004971286
- JAMES M. ET AL.: 'Synthesis and in vivo evaluation of a novel peripheral benzodiazepine receptor PET radioligand' BIOORG. MED. CHEM. vol. 13, no. 2, 2005, pages 6188 - 6194, XP005098157
- JANINE DOORDUIN ET AL: "[11C]-DPA-713 and [18F]-DPA-714 as New PET Tracers for TSPO: A Comparison with [11C]-(R)-PK11195 in a Rat Model of Herpes Encephalitis", MOLECULAR IMAGING AND BIOLOGY, SPRINGER-VERLAG, NE, vol. 11, no. 6, 28 March 2009 (2009-03-28) , pages 386-398, XP019754673, ISSN: 1860-2002, DOI: 10.1007/S11307-009-0211-6
- F. CHAUVEAU ET AL.: "Comprative Evaluation of the Translocator Protein Radioligands 11C-DPA-713, 18F-DPA-714, and 11C-PK11195 in a Rat Model of Acute Neuroinflammation", THE JOURNAL OF NUCLEAR MEDICINE, vol. 50, no. 3, March 2009 (2009-03), pages 468-476,

## Description

### TECHNICAL FIELD

This invention relates to compounds for imaging translocator protein (18 kDa) (TSPO) expression in a subject. This invention also relates to compounds for use in diagnosing neurodegenerative disorders in a subject.

### BACKGROUND ART

The translocator protein (18 kDa) (TSPO), formerly known as the peripheral benzodiazepine receptor (PBR), can form a trimeric complex with the adenine nucleotide carrier (ANC) (30 kDa) and the voltage-dependent anion channel (VDAC) (32 kDa) to constitute the mitochondrial permeability transition pore (MPTP). The TSPO is distinguished from the central benzodiazepine receptor (CBR) by its distinct structure, physiological functions and subcellular location on the outer membrane of the mitochondria. Although the TSPO has been implicated in numerous biological processes, some aspects of its physiological role remain unclear. Studies implicate the importance of the TSPO in the rate limiting step of steroid biosynthesis, immunomodulation, porphyrin transport, calcium homeostasis, and programmed cell death.

The TSPO is densely distributed in most peripheral organs including the lungs, heart and kidneys, yet it is only minimally expressed in the normal brain parenchyma. Following neuronal injury or infection, TSPO expression in the brain parenchyma is dramatically increased. *In vitro* autoradiography and immunohistochemistry has revealed that elevated TSPO binding in this region directly correlated with the appearance of activated microglia. Recently, *in vivo* positron emission tomography (PET) imaging in patients suffering from Alzheimer's disease (AD) and multiple sclerosis (MS) confirmed that TSPO binding in the brain parenchyma was confined to activated microglial cells.

Microglia are the principal immune effecter cells of the central nervous system (CNS). These macrophage-like immune cells are assumed to derive from monocytic lineage and their primary role lies in host defense and immune surveillance. They are highly sensitive to changes in their microenvironment and rapidly become activated in response to pathological events. For this reason the TSPO is believed to be intimately associated with initial inflammatory processes in the early stages of several neurodegenerative disorders.

A number of classes of TSPO ligands have been reported over the past few decades including the benzodiazepines (diazepam and Ro 5-4864), isoquinoline carboxamides (PK 11195), indoleacetamides (FGIN-1-27), phenoxyphenyl-acetamides (DAA1106), pyrazolopyrimides (DPA-713), benzothiazepines and imidazopyridines. Some other classes have also been developed, however, a more extensive range of ligands with varying binding properties and biological activity is required to better characterise the physiological and therapeutic roles of TSPO, its exact localisation and the anticipated existence of TSPO subtypes.

The isoquinoline carboxamide [¹¹C](R)-PK 11195 has been used as a pharmacological probe for studying the function and expression of TSPO. A number of PET studies conducted in patients with AD, MS and multiple system atrophy (MSA) has shown that measurement of TSPO *in vivo* with [¹¹C](*R*)-PK 11195 is feasible in the living brain. Although [¹¹C](*R*)-PK 11195 is regarded as the most widely used PET TSPO ligand it displays a poor signal to noise ratio and has demonstrated low brain permeability which ultimately decreases its sensitivity as a marker of microglial activation.

In 1998, the phenoxyphenyl-acetamide derivative, DAA1106, was reported as a highly selective and potent ligand for the TSPO (Chaki, S.; Funakoshi, T.; Yoshikawa, R.; Okuyama, S.; Okubo, T.; Nakazato, A.; Nagamine, M.; Tomisawa, K. European Journal of Pharmacology, 1999, 371, 197-204). Recently, DAA1106 was labelled with carbon-11 (11C) and used in PET studies to evaluate its *in vivo* kinetics in both rodent and primate brains (Zhang MR, Kida T, Noguchi J et al, [(11)C]DAA1106: radiosynthesis and in vivo binding to peripheral benzodiazepine receptors in mouse brain. Nucl Med Biol. 2003;30:513-519. Maeda J, Suhara T, Zhang MR et al. Novel peripheral benzodiazepine receptor ligand [11C]DAA1106 for PET: An imaging tool for glial cells in the brain. Synapse. 2004;52:283-291). The binding of [¹¹C]DAA1106 was shown to be four times greater than [¹¹C](*R*)-PK 11195 in the monkey occipital cortex, indicating its superior brain permeability. A fluorine-18 (¹⁸F) analogue of this compound has also been synthesised, namely [¹⁸F]FEDAA1106, and this analogue also displays similar binding characteristics *in vivo* to [¹¹C]DAA1106 (Zhang MR, Maeda J, Ogawa M et al. Development of a new radioligand, N-(5-fluoro-2-phenoxyphenyl)-N-(2-[18F]fluoroethyl-5-methoxybenzyl)acetamide, for pet imaging of peripheral benzodiazepine receptor in primate brain. J Med Chem. 2004;47:2228-2235). The binding of both [¹¹C]DAA1106 and [¹⁸F]FEDAA1106, however, appear to be irreversible and, in fact, their slow elimination from the brain indicates that they may not have suitable kinetics for quantitative analysis.

Thominiaux et al. (2006), Applied Radiation and Isotopes 64(5) 570-573 relates to the synthesis of peripheral benzodiazepine receptor ligand [¹¹C]DPA-713 using [¹¹C]methyl triflate. According to the results presented herein the synthesis of [¹¹C]DPA-713 by using [¹¹C]CH₃OTf improved the radiochemical yield when compared to the synthesis with [¹¹C] CH₃I. James et al. (2005), Bioorg. Med. Chem. 13(22) 6188-6194 relates to synthesis and in vivo evaluation of a peripheral benzodiazepine receptor PET radioligand. The results indicate that accumulation of [11C]1 in the baboon represents selective binding to the PBR. These exceptional in vivo binding properties suggest that [11C]1 may be useful for imaging the PBR in disease states. Furthermore, [11C]1 represents the first ligand of its pharmacological class to be labelled for PET studies and therefore has the potential to generate new information on the pathological role of the PBR in vivo.

Ryu JK et al, Neurobiology of Disease, 20 (2005) 550-561 reports that the TSPO ligand PK11195 reduces microglial activation and neuronal death in quinolinic acid-injected rat stratum. The results reported in this paper suggest that inflammatory responses from activated microglia are damaging to striatal neurons and thus pharmacological targeting of TSPO in microglia is likely to protect neurons in neurological disorders.

It would be advantageous to identify TSPO ligands with improved brain kinetics that can be used to image TSPO expression *in vivo,* as such ligands could be utilised to further study the cascade of biochemical events involved in the initial stages of several neurodegenerative disorders. It would also be advantageous to identify TSPO ligands with improved brain kinetics as such ligands have potential to serve as both diagnostic and therapeutic tools for neurodegenerative disorders.

### DISCLOSURE OF THE INVENTION

In a first aspect, the present invention provides a compound of formula (I): 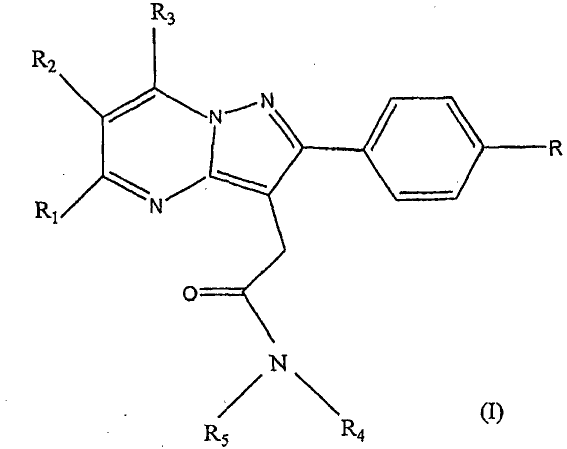 wherein:
R is H, halo, alkyl optionally substituted with halo, or alkoxy optionally substituted with halo;
R₁, R₂ and R₃ are each independently H or a hydrophobic group wherein the hydrophobic group is independently selected from C₁₋₆alkyl optionally substituted with halo, aryl optionally substituted with halo, NHC₁₋₆alkyl optionally substituted with halo, OC₁₋₆alkyl optionally substituted with halo, SC₁₋₆alkyl optionally substituted with halo, COOR₆ where R₆ is C₁₋₆alkyl optionally substituted with halo, (CH₂)ₙOR₆ where R₆ is C₁₋₆alkyl optionally substituted with halo, and n is an integer, and polyethers optionally substituted with halo; and
R₄ and R₅ are each independently alkyl optionally substituted with halo, or alkoxy optionally substituted with halo;
radiolabelled with a radioisotope selected from ¹⁸F, ¹²³I, ⁷⁶Br, ¹²⁴I and ⁷⁵Br, or a salt thereof.

As used herein, by a compound of formula (I) "radiolabelled" with ¹⁸F, ¹²³I, ⁷⁶Br, ¹²⁴I or ⁷⁵Br, it is meant that at least one of R₁, R₂, R₃, R₄ or R₅ in formula (I) is substituted with ¹⁵F, ¹²³I, ⁷⁶Br, ¹²⁴T or ⁷⁵Br. Typically one of R, R₁, R₂ or R₃ is substituted with ¹⁸F, ¹²³I, ⁷⁶Br, ¹²⁴I or ⁷⁵Br.

In a second aspect, the present invention provides a pharmaceutical composition comprising a compound of formula (I) radiolabelled with a radioisotope selected from ¹⁸F, ¹²³I, ⁷⁶Br, ¹²⁴I and ⁷⁵Br, or a pharmaceutically acceptable salt thereof, and a pharmaceutically acceptable carrier.

In a third aspect, the present invention provides a compound of formula (I), or a pharmaceutically acceptable salt thereof, for use in imaging translocator protein (18 kDa) in a subject.

Typically, when the compound of formula (I) is radiolabelled with ¹⁸F, ⁷⁶Br, ¹²⁴I or ⁷⁵Br, the image is obtained by positron emission tomography (PET) imaging. Typically, when the compound of formula (I) is radiolabelled with ¹²³I, the image is obtained by single photon emission computer tomography (SPECT) imaging.

In a fourth aspect, the present invention provides a compound of formula (I), or a pharmaceutically acceptable salt thereof, for use in diagnosing a neurodegenerative disorder in a subject.

### BRIEF DESCRIPTION OF THE DRAWINGS

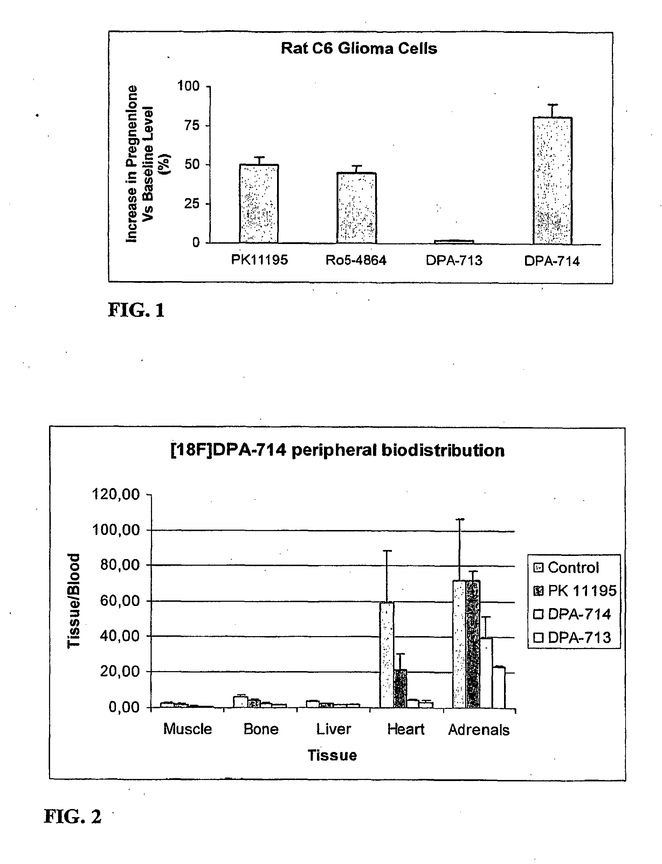

Figure 1 is a graph of the effect of the TSPO ligands PK11195, Ro5-4864, DPA-713 and DPA-714 on pregnenolone accumulation in C6 glioma rat cells. All compounds were used at the same concentration (40 µM); at the end of the incubation period (2h), the amount of pregnenolone was quantified by radio immunoassay (RIA). The values are the mean of at least three determinations.
Figure 2 is a graph of the peripheral distribution of [¹⁸F]DPA-714 in each of the four groups of QA lesioned rats described in Example 4; control (injected with radioligand only) and the three pre-treatment groups (PK 11195, DPA-713 and DPA-714).

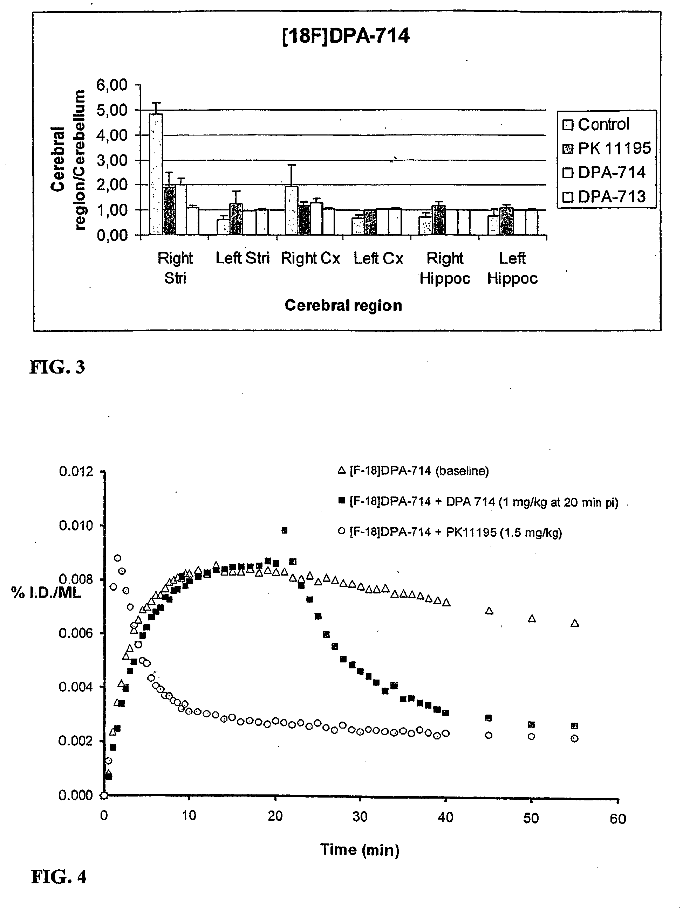

Figure 3 is a graph of the cerebral distribution of [¹⁸F]DPA-714 in the right striatum (Right Stri), left striatum (Left Stri), right frontal cortex (Right Cx), left frontal cortex (Left Cx), right hippocampus (Right Hippoc) and left hippocampus (Left Hippoc) in each of the four groups of QA lesioned rats described in Example 4; control (injected with radioligand only) and the three pre-treatment groups (PK 11195, DPA-713 and DPA-714).
Figure 4 shows time activity curves (TACs) depicting uptake of [¹⁸F]DPA-714 in the whole baboon brain during the 60 min PET scan time for the three studies described in Example 5 (baseline [¹⁸F]DPA-714, block [¹⁸F]DPA-714 + PK 11195 (1.5 mg/kg) and displacement [¹⁸F]DPA-714 + DPA-714 (1 mg/kg) study).

### DETAILED DESCRIPTION OF THE INVENTION

As used herein, the term "alkyl" refers to a straight chain, branched or mono- or polycyclic alkyl. Typically, the alkyl is a C₁ to C₃₀ alkyl, for example, a C₁ to C₆ alkyl. Examples of straight chain and branched alkyl include methyl, ethyl, propyl, isopropyl, butyl, isobutyl, sec-butyl, tert-butyl, pentyl, isopentyl, sec-pentyl, 1,2-dimethylpropyl, 1,1-dimethylpropyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl and 1,1,2-trimethylpropyl.

Examples of cyclic alkyl include cyclopropyl, cyclobutyl, cyclopentyl and cyclohexyl.

As used herein, the term "Alkoxy" refers to a group of the formula Oalkyl. Examples of alkoxy include methoxy, ethoxy, propoxy and butoxy.

As used herein, the term "alkenyl" refers to a straight chain, branched or cyclic alkenyl. Typically, the alkenyl is a C₂ to C₃₀ alkenyl, for example a C₂ to C₆ alkenyl. Examples of alkenyl include vinyl, allyl, 1-methylvinyl, butenyl, isobutenyl, 3-methyl-2-butenyl, 1-pentenyl, cyclopentenyl, 1-methylcyclopentenyl, 1-hexenyl, 3-hexenyl, cyclohexenyl, 1-heptenyl, 3-heptenyl, 1-octenyl, cyclooctenyl, 1-nonenyl, 2-nonenyl, 3-nonenyl, 1-decenyl, 3-decenyl, 1,3-butadienyl, 1,4-pentadienyl, 1,3-cyclopentadienyl, 1,3-hexadienyl, 1,4-hexadienyl, 1,3-cyclohexadienyl, 1,4-cyclohexadienyl, 1,3-cycloheptadienyl, 1,3,5-cycloheptatrienyl and 1,3,5,7-cyclooctatetraenyl.

As used herein, the term "alkynyl" refers to a straight chain, branched or cyclic alkynyl. Typically, the alkynyl is a C₂ to C₃₀ alkynyl, for example, a C₂ to C₆ alkynyl.

As used herein, the term "aryl" refers to a radical of a single, polynuclear, conjugated or fused aromatic hydrocarbon or aromatic heterocyclic ring system. Examples of aryl include phenyl, naphthyl and furyl. When the aryl comprises a heterocyclic aromatic ring system, the heterocyclic aromatic ring system may contain 1 to 4 heteroatoms independently selected from N, O and S.

As used herein, the term "halo" refers to a halogen radical, e.g. fluoro, chloro, bromo or iodo. As used herein, a reference to a group "optionally substituted with halo" means the group may be substituted with one or more halogen substituents.

The present inventors have surprisingly found that the compounds of formula (I) radiolabelled with a radioisotope selected from ¹⁸F, ¹²³I, ⁷⁶Br, ¹²⁴I and ⁷⁵Br are selective TSPO ligands, and can be used as accurate *in vivo* markers of TSPO and therefore microglial activation. These radiolabelled compounds can therefore be used to study neuropathological events in a number of neurodegenerative disorders, and can be used as a tool for diagnosis of such disorders and for monitoring the progression of such disorders.

A number of classes of TSPO ligands have been described in the literature. A compound which is effective as a therapeutic drug is not necessarily a compound that can be radiolabelled and used for imaging. Indeed, many drugs that are used therapeutically are not selective for a specific target and may interact with several targets to produce a therapeutic effect. Further, many therapeutic drugs do not have affinity that is in the nM range normally used for imaging, but have affinity in the µM range. In addition, the metabolism and lipophilicity of a therapeutic drug, particularly when administered at tracer levels for imaging, may make the drug unsuitable for use for imaging.

The present inventors have surprisingly found that compounds of formula (I) radiolabelled with a radioisotope selected from ¹⁸F, ¹²³I, ⁷⁶Br, ¹²⁴I and ⁷⁵Br can be used to image TSPO and therefore microglial activation in a subject. The compounds of formula (I) radiolabelled with ¹⁸F, ¹²³I, ⁷⁶Br, ¹²⁴I or ⁷⁵Br are selective ligands for TSPO and have high affinity for TSPO.

The compounds of formula (I) radiolabelled with a radioisotope selected from ¹⁸F, ¹²³I, ⁷⁶Br, ¹²⁴I and ⁷⁵Br form salts, and salts of such compounds are encompassed by the present invention. The salts are preferably pharmaceutically acceptable, but it will be appreciated that non-pharmaceutically acceptable salts also fall within the scope of the present invention. Examples of pharmaceutically acceptable salts include salts of pharmaceutically acceptable cations such as sodium, potassium, lithium, calcium, magnesium, ammonium and alkylammonium; acid addition salts of pharmaceutically acceptable inorganic acids such as hydrochloric, orthophosphoric, sulphuric, phosphoric, nitric, carbonic, boric, sulfamic and hydrobromic acids; or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulphonic, trihalomethanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

In formula (I), R is typically C₁₋₆ alkyl or C₁₋₆ alkoxy, wherein the C₁₋₆ alkyl or C₁₋₆ alkoxy may be optionally substituted with halo.

Typically, R₁ and R₃ are each a group other than H. When R₁, R₂ or R₃ is a hydrophobic group, the hydrophobic group is C₁₋₆ alkyl optionally substituted with halo, aryl optionally substituted with halo, NHC₁₋₆ alkyl optionally substituted with halo, OC₁₋₆ alkyl optionally substituted with halo, SC₁₋₆ alkyl optionally substituted with halo, COOR₆ where R₆ is C₁₋₆ alkyl optionally substituted with halo, (CH₂)ₙOR₆ where R₆ is C₁₋₆ alkyl optionally substituted with halo and n is an integer (e.g. 1, 2, 3, 4, 5 or 6), or a polyether optionally substituted with halo. When R₁, R₂ or R₃ is a polyether optionally substituted with halo, the polyether may, for example, be a group of the formula - (O(CH₂)ₐ)_{b} (CH₂)_{c}CH₃, where a is 1,2 or 3, b is 2, 3, 4 or 5, and c is 0, 1, 2, 3, 4 or 5, optionally substituted with halo.

Various compounds of formula (I) are described in Selleri et al, "2-Arylpyrazolo[1,5-a]pyrimidin-3-yl Acetamides. New Potent and Selective Peripheral Benzodiazepine Receptor Ligands", Bioorganic & Medicinal Chemistry 9 (2001) 2661-2671 ("*Selleri et al* (2001)"). That document describes the preparation of certain specific compounds of formula (I) (compounds 3f to 3y described in that document) and discloses that those compounds have affinity and selectivity for TSPO (referred to as PBR in that document). Whilst some of the specific compounds described in that document have some affinity for CBR, each of compounds 3f to 3y described in that document is much more selective for TSPO than CBR.

As discussed in *Selleri et al* (2001), the compounds of formula (I) referred to in that document can be prepared in a three step procedure starting from the appropriate aroylacetonitrile. In the process described, the aroylacetonitrile was reacted in alkaline medium with *N,N*-diethylchloroacetamide, with the resultant tar purified by means of column chromatography to isolate the *N,N*-diethylbutanamide. The *N,N-*diethylbutanamide was reacted at reflux in ethanol with hydrazine hydrate, in the presence of acetic acid, to give the corresponding *N,N-*diethyl-(3-amino-5-arylprazol-4-yl)acetamide. The *N,N-*diethyl-(3-amino-5-arylpyrazol-4-yl)acetamide was then condensed with a suitable electrophilic reagent (4,4-dimethoxy-2-butanone, 1,1,3,3-tetraethoxypropane, 2,4-pentanedione, 1-trifluoromethyl-2,4-pentanedione, 1,5-trifluoromethyl-2,4-pentanedione, 1-phenyl-1,3-butanedione, 3-methyl-2,4-pentanedione, ethyl 2-acetyl-3-oxo butanoate, ethyl 2-acetyl-3-ethoxyacrylate, phenylmalondialdehyde or 1-phenyl-3-dimethylamino-2-propen-1-one) to close the pyrimidine ring, thus forming the relevant compound of formula (I).

Another method of preparing various compounds of formula (I) is described in Selleri, S.; Gratteri, P.; Costagli, C.; Bonaccini, C.; Costanzo, A.; Melani, F.; Guerrini, G.; Ciciani, G.; Costa, B.; Spinetti, F.; Martini, C.; Bruni, F. Bioorganic and Medicinal Chemistry, 2005, 13, 4821-4834 ("*Selleri et al* (2005)"). In the method described in that document, benzoylacetonitrile was reacted in basic medium (LiOH·H₂O) with iodoacetric acid or ethyl iodoacetate to give the corresponding acid or ester (1a and 1b) respectively. The intermediates 1a and 1b were reacted in ethanol at reflux with hydrazine hydrate, in the presence of acetic acid, to give the corresponding 3-amino pyrazoles (2a and 2b). The compounds 2a and 2b were condensed with 2,4-pentanedione to close the pyrimidine ring resulting in acid 3a and ester 3b. The acid 3a was converted to a mixed anhydride (with ethyl chloroformate) and this intermediate reacted with an amide to produce the relevant compound of formula (I).

The compounds of formula (I) described in *Selleri et al* (2001) and *Selleri et al* (2005) may be prepared by the processes described in those documents, or by other processes known to persons skilled in organic chemistry synthesis. As will be apparent to a person skilled in the art, other compounds of formula (I) can be prepared by similar processes to the processes described in *Selleri et al* (2001) and *Selleri et al* (2005).

A compound of formula (I) can be radiolabelled with ¹⁸F, ¹²³I, ⁷⁶Br, ¹²⁴I or ⁷⁵Br by standard techniques known in organic chemistry for modifying an organic compound to replace a hydrogen or halo group in the compound with ¹⁸F, ¹²³I, ⁷⁶Br, ¹²⁴I or ⁷⁵Br. Alternatively, compounds of formula (I) radiolabelled with a radioisotope selected from ¹⁸F, ¹²³I, ⁷⁶Br, ¹²⁴I and ⁷⁵Br may be prepared by incorporating ¹⁸F, ¹²³I, ⁷⁶Br, ¹²⁴I or ⁷⁵Br as a substituent in one of the starting materials or in an intermediate used in the synthesis of the compound of formula (I).

A compound of formula (I) radiolabelled with ¹⁸F, ¹²³I, ⁷⁶Br, ¹²⁴I or ⁷⁵Br may, for example, be prepared by preparing a compound having the formula (I) defined above, but in which one of R₁, R₂, R₃, R₄ or R₅ is substituted with a leaving group, such as tosylate, mesylate, Br or I, that allows an aliphatic nucleophilic substitution reaction to occur at the leaving group, and then subjecting the compound to conditions under which an aliphatic nucleophilic substitution reaction occurs to replace the leaving group with ¹⁸F, ¹²³I, ⁷⁶Br, ¹²⁴I or ⁷⁵Br. For example, when the leaving group is Br or tosylate, the compound may be reacted with the [¹⁸F]-kryptofix-K222 complex in acetonitrite at about 80°C for 10 minutes to form a compound of formula (I) radiolabelled with ¹⁸F. Compounds of formula (I) radiolabelled with ¹²¹I, ⁷⁶Br, ¹²⁴I or ⁷⁵Br may also be formed by forming a compound having the formula (I) defined above, but in which one of R, R₁, R₂, R₃, R₄ or R₅ is substituted with a stannyl, silyl or halogen (the halogen substituent is usually different to the radioisotope), and subjecting the compound to an electrophilic substitution reaction in acetic media using an oxidising agent such as chloramine-T to form a compound of formula (I) radiolabelled with ¹²³I, ⁷⁶Br, ¹²⁴I or ⁷⁵Br. In some embodiments, this reaction may be carried out at room temperature, and in other embodiments, the reaction mixture is heated to about 80 °C to 100 °C. A compound of formula (I) as defined above, but in which one of R, R₁, R₂, R₃, R₄ or R₅ is substituted with a leaving group, may be prepared by analogous processes to the processes for preparing compounds of formula (I) described in *Selleri et al* (2001) or *Selleri et al* (2005) but in which an appropriate reactant is substituted with the leaving group. Alternatively, a compound of formula (I) may be modified by reactions known in organic chemistry to introduce a leaving group as a substituent on one of R, R₁, R₂, R₃, R₄ or R₅.

The compound of formula (I) is radiolabelled with ¹⁸F (half-life 110 minutes), ¹²³I (half-life 13.2 hours), ⁷⁶Br (half-life 16.2 hours), ¹²⁴I (half-life 4.2 days) or ⁷⁵Br (half-life 1.6 hours). Typically, the compound of formula (I) is radiolabelled with ¹⁸F. Compounds of formula (I) radiolabelled with ¹⁸F, ¹²³I, ⁷⁶Br, ¹²⁴I or ⁷⁵Br are more practical in a clinical sense for imaging than compounds radiolabelled with radioisotopes having a significantly shorter half-life, as multiple scans can be performed on one day. In addition, hospitals/organisations that do not have a cyclotron on site can use such radioligands, as the radioligands can be prepared offsite and transported to the hospital/organisation with no significant loss of activity during transportation. In addition, longer scans (e.g. 180 minutes) can be undertaken with compounds labelled with ¹⁸F, ¹²³I, ⁷⁶Br, ¹²⁴I or ⁷⁵Br making them more appropriate for the study of most biological processes.

Compounds of formula (I) radiolabelled with ¹⁸F, ¹²³I, ⁷⁶Br, ¹²⁴I or ⁷⁵Br have high affinity and selectivity for TSPO, and can be used for imaging TSPO in a subject. Accordingly, compounds of formula (I) radiolabelled with ¹⁸F, ¹²³I, ⁷⁶Br, ¹²⁴I or ⁷⁵Br can be used to study TSPO in a subject.

In a subject having a neurodegenerative disorder, TSPO expression in the brain parenchyma is dramatically increased compared to a subject not having a neurodegenerative disorder. Accordingly, the compounds of formula (I) radiolabelled with ¹⁸F, ¹²³I, ⁷⁶Br, ¹²⁴I or ⁷⁵Br can be used to study neurodegenerative disorders and can be used to diagnose and monitor the progression of neurodegenerative disorders. Neurodegenerative disorders that can be studied, diagnosed or monitored using these compounds include Alzheimer's disease, multiple sclerosis, Parkinson's disease, Huntington's disease, multiple system atrophy, epilepsy, encephalopathy, stroke and brain tumours. Each of these disorders is associated with neuronal injury or infection.

Disclosed is that a compound of formula (I) radiolabelled with a radioisotope selected from ¹⁸F, ¹²³I, ⁷⁶Br, ¹²⁴I and ⁷⁵Br or a pharmaceutically acceptable salt thereof can be administered to the subject. When the compound of formula (I) is radiolabelled with ¹⁸F, ⁷⁶Br, ¹²⁴I or ⁷⁵Br, the image of the location of the radioisotope in the subject, and therefore the location of TSPO in the subject, may be obtained by positron emission tomography (PET) imaging using conventional techniques known the art. When the compound is radiolabelled with ¹²³I, the image of the location of the radioisotope in the subject may be obtained by SPECT imaging using conventional techniques known in the art. Typically for both PET and SPECT imaging, the data is acquired using conventional dynamic or list mode acquisition technique, commencing immediately after administration of the compound of formula (I) radiolabelled with ¹⁸F, ¹²³I, ⁷⁶Br, ¹²⁴I or ⁷⁵Br or pharmaceutically acceptable salt thereof, and continuing for about 40 minutes or longer. At the completion of data acquisition, the data is typically processed to provide a time-series of 3D reconstructions, each depicting the distribution of the radioisotope in the body at a particular point in time.

Disclosed is that typically, the compound of formula (I) radiolabelled with ¹⁸F, ¹²³I, ⁷⁶Br, ¹²⁴I or ⁷⁵Br or pharmaceutically acceptable salt thereof can be administered parenterally. Typically, the compound of formula (I) radiolabelled with ¹⁸F, ¹²³I, ⁷⁶Br, ¹²⁴I or ⁷⁵Br or pharmaceutically acceptable salt thereof can be administered parenterally by intravenous injection or infusion. Typically the compound of formula (I) radiolabelled with ¹⁸F, ⁷⁶Br, ¹²⁴I or ⁷⁵Br or pharmaceutically acceptable salt thereof can be administered at a dose in the range of about 5 to 20 mCi (185-740 MBq).

Disclosed is that typically, the compound of formula (I) radiolabelled with ¹⁸F, ¹²³I, ⁷⁶Br, ¹²⁴I or ⁷⁵Br or pharmaceutically acceptable salt thereof can be administered by administering a pharmaceutical composition comprising the compound of formula (I) radiolabelled with¹⁸F, ¹²³I, ⁷⁶Br, ¹²⁴I or ⁷⁵Br, or pharmaceutically acceptable salt thereof, and a pharmaceutically acceptable carrier.

Disclosed is that preparations for parenteral administration are typically in the form of a sterile aqueous or non-aqueous solution, suspension or emulsion. Examples of suitable non-aqueous solvents are propylene glycol, polyethylene glycol, vegetable oils such as olive oil, and injectable organic esters such as ethyl oleate. Suitable aqueous carriers include water and alcoholic/aqueous solutions, emulsions or suspensions, including saline and buffered media. Suitable parenteral vehicles include sodium chloride solution.

Generally, the terms "treating", "treatment" and the like are used herein to mean affecting a subject to obtain a desired pharmacological and/or physiological effect. The effect may be prophylactic in terms of completely or partially preventing a disease or disorder or sign or symptom thereof, and/or may be therapeutic in terms of a partial or complete cure of a disease or disorder. "Treating" as used herein covers any treatment of, or prevention of, disease or disorder in a vertebrate, a mammal, particularly a human, and includes: (a) preventing the disease or disorder from occurring in a subject that may be predisposed to the disease or disorder, but has not yet been diagnosed as having the disease or disorder; (b) inhibiting the disease or disorder, i.e., arresting the development of the disease or disorder; or (c) relieving or ameliorating the effects of the disease or disorder, i.e. causing regression of the effects of the disease or disorder.

### EXAMPLES

Parts of the Examples not pertaining to the invention as claimed are for illustrative or comparative purposes only.

### EXAMPLE 1 - Synthesis of DPA-714 and [¹⁸F]DPA-714

### 1. Synthesis of DPA-714

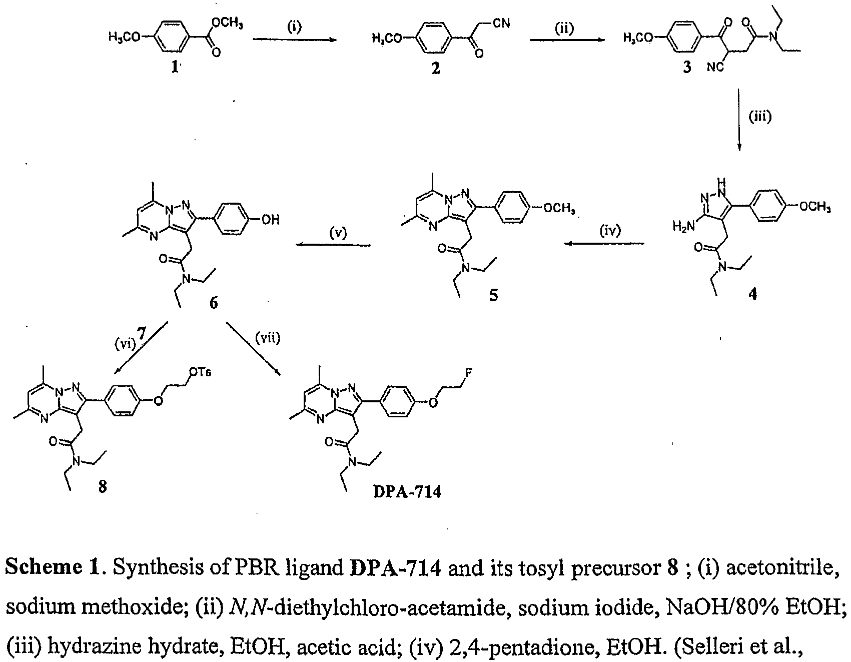

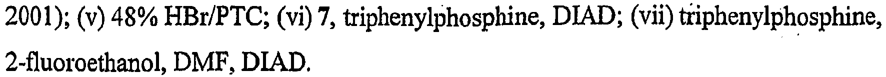

### 3-(4-Methoxy-phenyl)-3-oxo-propionitrile (2)

A mixture of methyl 4-methoxybenzoate (30 g, 181 mmol) and sodium methoxide (9.75 g, 181 mmol) was heated at 80 °C under an argon atmosphere with continuous stirring until homogenous. Acetonitrile (16.5 ml, 313 mmol) and chlorobenzene (19 ml) was added dropwise to the mixture. The reaction was heated at 90-100 °C for 24 hours with continuous stirring. After the mixture was cooled to ~ 0 °C and treated with ice water (~50 ml) and diethyl ether (~200 ml), it was shaken until the solid material dissolved. The aqueous layer was separated from the organic layer and acidified to pH 2 with dilute H₂SO₄. Following the addition of diethyl ether, the organic layer was extracted, dried over anhydrous Na₂SO₄ and evaporated to dryness. The resulting yellow solid was dissolved in CHCl₃ and washed with saturated NaHCO₃ aqueous solution (5 x 100 ml) to remove benzoic acid. The organic layer was dried over anhydrous NaSO₄ and evaporated to dryness. The solid was purified by washing with petroleum ether which yielded **2** (2.91 g, 9 %) as fine, light yellow crystals; mp: 132-137 °C; ¹H n.m.r. (CDCl₃, 300 MHz) δ 3.89 (s, 3H, OCH₃), 4.03 (s, 2H, CH₂), 6.97 (d, *J* = 9.0, 2H, Ph), 7.90 (d, *J* = 9.0, 2H, Ph).

### 3-Cyano-N,N-diethyl-4-(4-metkoxy-phenyl)-4-oxo-butyramide (3)

A mixture of 2 (2.0 g, 11.4 mmol), N',N-diethylchloroacetamide (1.7 g, 11.4 mmol) and NaI (5.1 g, 34 mmol) were added to a solution of NaOH (0.5 g, 12.5 mmol) in 80% EtOH (80 ml) whilst being stirred continuously. The mixture was stirred at room temperature for 7 hours and monitored by t.l.c. Once the reaction was complete it was allowed to cool and was filtered to remove the inorganic material. The filtrate was concentrated and the residue was purified by column chromatography (CH₂Cl₂ as eluent) to yield 3 (2.1 g, 64 %) as a dark yellow oil; ¹H n.m.r. (CDCl₃, 300 MHz) δ 1.06-1.30 (m, 6H, N(CH₂CH₃)₂), 2.85 (dd, *J* = 4.5, 16.2 Hz, 1H, CH₂), 3.21-3.43 (m, 5H: 4H, N(CH₂CH₃)₂ : 1H, CH₂), 3.90 (s, 3H, OCH₃), 4.89-5.02 (m, 1H, CH), 6.98 (d, *J* = 8.7, 2H, Ph), 8.05 (d, *J* = 9.0, 2H, Ph). Mass Spectrum: CI, m/z 289 (M + 1).

### 2-[3-Amino-5-(4-methoxy-phenyl)-1H-pyrazol-4-yl]-N,N-diethylacetamide (4)

Hydrazine hydrate (0.73 g, 14.6 mmol) and acetic acid (0.73 ml) was added to a solution of **3** (2.1 g, 7.3 mmol) in EtOH (37 ml). The mixture was heated at reflux for 4 hours and monitored by t.l.c. Once the reaction was complete, it was allowed to cool to room temperature. The solution was evaporated to dryness and the residue was purified by silica gel column chromatography (CH₂Cl₂/MeOH, 10:1 v/v, as eluent). The purified product was re-dissolved in CH₂Cl₂ and washed with saturated NaHCO₃ aqueous solution (4 x 20 ml) to remove acetic acid. This afforded **4** (1.52 g, 68 %) as yellow crystals; mp: 154.5-157.5 °C; ¹H n.m.r. (CDCl₃, 300 MHz) δ 0.90-1.10 (m, 6H, N(CH₂CH₃)₂), 3.04-3.33 (m, 4H, N(CH₂CH₃)₂), 3.50 (s, 2H, CH₂), 3.85 (s, 3H, OCH₃), 6.98 (d, *J* = 8.7, 2H, Ph), 7.32 (d, *J* = 9.0, 2H,Ph).

### N,N-Diethyl-2-[2-(4-methoxy-phenyl)-5,7-dimethylpyrazolo[1,5-a]pyrimidin-3-yl]-acetamide (5)

2,4-Pentanedione (0.4 g, 4 mmol) was added to a solution of **4** (1.2 g, 4 mmol) in EtOH (20 ml). The mixture was heated at reflux for 12 hours. The reaction mixture was allowed to cool and the solvent was evaporated to dryness. The residue was purified by silica gel column chromatography (CHCl₃/MeOH, 40:1 v/v as eluent) which yielded **5** (1.37 g, 93 %) as pale yellow crystals; mp: 120.5-123.5 °C; ¹H n.m.r. (CDCl₃, 300 MHz) δ 1.09-1.22 (m, 6H, N(CH₂CH₃)₂), 2.54 (s, 3H, 5-CH₃), 2.74 (s, 3H, 7-CH₃), 3.39-3.51 (m, 4H, N(CH₂CH₃)₂), 3.85 (s, 3H, OCH₃), 3.91 (s, 2H, CH₂), 6.51 (s, 1H, H-6), 6.98 (d, *J* = 9.0, 2H, Ph), 7.76 (d, *J* = 9.0, 2H, Ph).

### N,N-Diethyl-2-[2-(4-hydroxy-phenyl)-5,7-dimethyl-pyrazolo[1,5-a]pyrimidin-3-yl]-acetamide (6)

A solution of **5** (0.43 g, 1.16 mmol), hexadecyl tributyl phosphonium bromide (0.06 g, 0.116 mmol) and 45 % HBr (6 ml) was heated at 100 °C for 7 hours under constant stirring. The reaction mixture was basified to pH 8-9 using NaHCO₃ and extracted with CH₂Cl₂. The organic layer was collected and dried over anhydrous NaSO₄. The solvent was removed under vacuum and the residue was purified by column chromatography (CHCl₃/MeOH, 40:1 v/v as eluent) to yield **6** (220 mg, 54%) as ivory crystals; mp: 242.5-247 °C; ¹H n.m.r. (CDCl₃, 300 MHz) δ 1.06-1.18 (m, 6H, N(CH₂CH₃)₂), 2.54 (s, 3H, 5-CH₃), 2.73 (s, 3H, 7-CH₃), 3.34-3.51 (m, 4H, N(CH₂CH₃)₂), 3.96 (s, 2H, CH₂), 6.49 (s, 1H, H-6), 6.79-6.82 (d, *J* = 8.7,2H, Ph), 7.61-7.64 (d, *J* = 8.4, 2H, Ph); (Found, C, 66.35; H, 6.71; N, 15.38. C₂₀H₂₄N₄O₂.1/2H₂O requires C, 68.16; H, 6.86; N, 15.90%). Mass Spectrum: CI, m/z 353 (M + 1).

### Toluene-4-sulfonic acid 2-hydroxy-ethyl ester (7)

Fresh silver oxide (350 mg, 1.5 mmol), p-toluenesulfonyl chloride (210 mg, 1.1 mmol) and potassium iodide (33 mg, 0.2 mmol) were added to a stirred solution of 1,2-ethanediol (62 mg, 1 mmol) in dichloromethane (10 ml). The reaction mixture was stirred at room temperature for 1 hour, filtered through a small pad of celite and washed with ethyl acetate. The solvent was removed and the crude product was purified by column chromatography (CH₂Cl₂/MeOH, 40:1 v/v as eluent) to yield the monotosylate product **7** as transparent oil in 46% yield.¹H n.m.r. (CDCl₃, 300 MHz) δ: 7.81 (d, 2H, *J* = 8.1), 7.36 (d, 2H, *J* = 8.1), 4.15(d, 2H, *J* = 9.0), 3.82 (d, 2H, *J* = 9.0), 2.46 (s, 3H).

### Toluene-4-sulfonic acid 2-[4-(3-diethylcarbamoylmethyl-5,7-dimethyl-pyrazolo-[1,5-a]pyrimidin-2-yl)-phenoxy]-ethyl ester (8)

Diisopropyl azodicarboxylate (DIAD, 0.48 ml, 2.4 mmol) was added to a solution of 6 (400 mg, 1.1 mmol) triphenylphosphine (637 mg, 2.4 mmol) and 2-hydroxyethyl tosylate (525 mg, 2.4 mmol) in dry THF (10 ml). The reaction mixture was stirred for 20 hours at room temperature and evaporated to dryness. The residue was purified by silica gel column chromatography (CHCl₃/MeOH, 80:1 v/v as eluent) to yield **8** as light yellow crystals in 85% yield. ¹H n.m.r. (CDCl₃, 300 MHz) δ: 7.83 (d, 2H, *J* = 8.1), 7.74 (d, 2H, *J* = 9.0), 7.35 (d, 2H, *J* = 8.1), 6.86 (d, 2H, *J* = 9.0), 6.52 (s, 1H), 4.37 (d, 2H, *J* = 4.8), 4.19 (d, 2H, *J* = 4.8), 3.92 (s, 2H), 3.39-3.52 (m, 4H), 2.74 (s, 3H), 2.56 (s, 3H), 2.45 (s, 3H), 1.08-1.23 (m, 6H); (Found, C, 63.37; H, 5.96; N, 9.89. Mass Spectrum: CI, m/z 551 (M + 1).

### N,N-Diethyl-2-{2-[4-(2-fluoro-ethoxy)-phenyl]-5,7-dimethyl-pyrazolo[1,5-a]pyrimidin-3-yl}-acetamide (DPA-714)

Diisopropyl azodicarboxylate (DIAD, 190 mg, 0.94 mmol) was added to a solution of **6** (150 mg, 0.43 mmol), triphenylphosine (274 mg, 0.94 mmol) and 2-fluoroethanol (60 mg, 0.94 mmol) in dry DMF (6 ml). The reaction mixture was stirred at room temperature for 48 hours and then evaporated for 48 hours and then evaporated to dryness. The residue was purified by silica gel column chromatography (CHCl₃/MeOH, 80:1 v/v as eluent) to yield DPA-714 as pale yellow crystals in 47% yield. ¹H n.m.r. (CDCl₃, 300 MHz) δ**:** 7.78 (d, 2H, *J* = 9.0 Hz), 6.52 (s, 1H), 7.01 (d, 2H, *J* = 9.0 Hz), 4.78 (dt, 2H, *J* = 4,47 Hz), 4.26 (dt, 2H, *J* = 4,28 Hz), 3.93 (s, 2H), 3.39-3.51 (m, 4H), 2.75 (s, 3H), 2.56 (s, 3H), 1.09-1.27 (m, 6H); (Found, C, 66.70; H, 6.60; N, 13.14. Mass Spectrum: CI, m/z 399 (M + 1).

### 2. Radiosynthesis of [¹⁸F]DPA-714

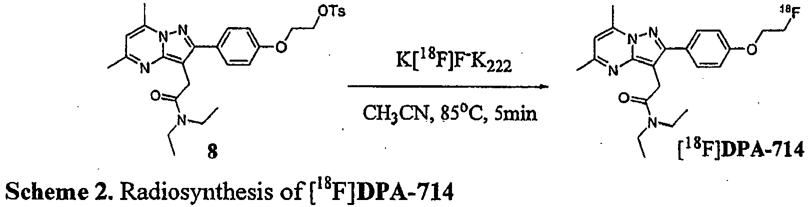

**Radioisotope production.** No carrier added-aqueous [¹⁸F]fluoride ion was produced on a PETtrace cyclotron (GE Healthcare, Sweden), by irradiation of a 0.8 mL water target using a 16.5 MeV proton beam on 95% enriched [¹⁸O]-H₂O by the [¹⁸O(p,n)¹⁸F] nuclear reaction.

**Preparation of [¹⁸F]-kryptofix-K222.** [¹⁸F]Fluoride in [¹⁸O] enriched-H₂O was transferred to the GE TRACERlab MX_{FDG} synthesiser and passed through an anion exchange resin (Sep-Pak Waters Accell^{™} Light QMA cartridge in the carbonate form, prepared by washing with 10 mL 0.5 M K₂CO₃ and then rinsed with 10 mL of water) under vacuum. Trapped [¹⁸F]fluoride ions were then eluted from the Sep-Pak cartridge and transferred to the reactor vessel using an eluent solution containing K₂CO₃ (7 mg in 300 µL of pure water), 300 µL of acetonitrile and 22 mg of Kryptofix 222 (K222: 4,7,13,16,21,24-hexaoxa-1,10-diazabicyclo [8.8.8] hexacosan). Aliquots of acetonitrile were added and the reaction mixture evaporated to dryness after each addition. (3 times : 80 µL, each time). The evaporation was carried out at 95°C under nitrogen flow and vacuum.

**Preparation and formulation of [¹⁸F]DPA-714.** Tosylate precursor (7) was dissolved in 3 mL of acetonitrile and added to the dry [¹⁸F]-kryptofix-K222 complex. The mixture was allowed to react at 85°C for 5 minutes. Upon completion the reaction mixture was diluted with water for injection Waters for Injections BP (WFI BP) and passed through a tC-18 Sep-Pak cartridge. The reactor vessel was rinsed with WFI and again passed through the tC 18 Sep-Pak cartridge. The tCl8 trapped radiolabelled product was rinsed a further three times with WFI (40mL total). The product was then eluted from the tC18 Sep-Pak cartridge with EtOH (2mL) and WFI (3mL). The resulting solution was passed though a 0.22 µm Millipore CATHIVEX non-pyrogenic sterile filter to remove particulate material before HPLC purification. The crude mixture was then injected onto a HPLC Waters XTerra RP C-18 10µm (7.8 x 300 mm) semi-preparative reversed-phase column. Using a mobile phase of 0.1 M NH₄Ac : CH₃CN (pH=10) : (60/40, v:v), and with a flow rate of 4.0 mL/min, the retention time (t_{R}) of [¹⁸F]DPA-714 was 12.42 min. The radioactive fraction corresponding to [¹⁸F]DPA-714 was collected and evaporated under vacuum. The residue was reconstituted in WFI BP (4 mL) and filtered through a sterile 13 mm Millipore GV 0.22 µm filter into a sterile pyrogen free evacuated vial. This afforded [¹⁸F]DPA-714 in 10% (n=6) non-decay corrected radiochemical yield.

**Quality control of [¹⁸F]DPA-714.** For determination of specific radioactivity and radiochemical purity, an aliquot of the final solution of known volume and radioactivity was injected onto an analytical reversed-phase HPLC column (Waters XTerra C18 5µm (4.6 x 150 mm). A mobile phase of 0.1 M NH₄Ac : CH₃CN (pH=10) : (50:50; v:v) at a flow rate of 2.0 mL/min was used to elute [¹⁸F]DPA-714 with a retention time (t_{R}) of 2.23 min. The area of the UV absorbance peak measured at 254 nm corresponding to the carrier product was measured (integrated) on the HPLC chromatogram and compared to a standard curve relating mass to UV absorbance. Radiochemical and chemical purity was greater than 98% and specific activity was 1680 GBq/µmol.

### EXAMPLE 2 - In vitro binding affinity of DPA-714

For binding studies, mitochondria were prepared as described previously (Trapani G, Franco M, Ricciardi L, et al. Synthesis and binding affinity of 2-phenylimidazo[1,2-alpha]pyridine derivatives for both central and peripheral benzodiazepine receptors. A new series of high-affinity and selective ligands for the peripheral type. Journal of Medicinal Chemistry. 1997;40:3109-3118 and Campiani G, Nacci V, Fiorini I, et al. Synthesis, Biological Activity, and SARs of Pyrrolobenzoxazepine Derivatives, a New Class of Specific "Peripheral-Type" Benzodiazepine Receptor Ligands. Journal of Medicinal Chemistry. 1996;39:3435-3450) with minor modifications as described below, from kidneys of Male Wistar rats killed by cervical dislocation. Kidneys were homogenized in 20 volumes of ice-cold 50 mM Tris/HCl, pH 7.4,0.32 M sucrose and 1 mM EDTA (buffer A), containing protease inhibitors (160 µg/mL benzamidine, 200 µg/mL bacitracine and 20 µg/mL soybean trypsin inhibitor) with a Teflon pestle in a glass homogenizer and centrifuged at 600g for 10 min at 4 °C. The resulting supernatant was centrifuged at 10,000g for 10 min at 4°C. The pellet was then resuspended in 20 volumes of ice-cold buffer A, and centrifuged again at 10,000g for 10 min at 4 °C. The crude mitochondrial pellet was frozen at -20°C until the time of assay or incubated with 0.6 nM [³H]PK11195 in 50 mM Tris/HCl, pH 7.4 (buffer B), with a range of concentrations of the tested compounds (0.1 nM to 10 µM) in a total volume of 0.5 mL for 90 min at 4 °C. The incubation was terminated by dilution to 5 mL with ice-cold buffer B, followed immediately by rapid filtration through glass fiber Whatman GF/C filters. The filters were then washed (2 5 mL) with buffer B and the amount of radioactivity retained on the filters was determined by Packard 1600 TR liquid scintillation counter at 66% efficiency. Non-specific binding was estimated in each case in the presence, respectively, of unlabeled 1 µM PK 11195. The IC₅₀ values were determined and Kᵢ values were derived according to the equation of Cheng and Prusoff (Cheng Y-C, Prusoff WH. Relationship between the inhibition constant (KI) and the concentration of inhibitor which causes 50 per cent inhibition (150) of an enzymatic reaction. Biochemical Pharmacology. 1973;22:3099-3108). Protein concentration was estimated by the method of Lowry *et al* (Lowry OH, Rosebrough NJ, Farr AL, Randall RJ. Protein measurement with the folin phenol reagent. Journal of Biological Chemistry. 1951;193:265-275) with bovine serum as standard.

The affinity of DPA-714 for the TSPO was evaluated by membrane binding assay using [³H]PK 11195 as the radioligand and rat kidney tissue as the receptor source. To ensure the selectivity of DPA-714, its binding to the CBR was assessed using [³H]Ro 15-1788 and rat brain tissue. The results are shown in Table 1. As shown in Table 1, the affinity of DPA-714, *K*ᵢ = 7.0 nM, was not as high as DPA-713, *K*ᵢ = 4.7 nM, yet was comparably higher than PK 11195, *K*ᵢ = 9.3 nM in the same assay. All three TSPO ligands, DPA-714, DPA-713 and PK 11195 displayed negligible affinites for the CBR.

**Table 1.** Affinities of ligands for TSPO and CBR using [³H]PK 11195 and [³H]Ro 15-1788 as the radioligand and rat kidney membranes and rat brain tissue as receptor source respectively. Log D values for each ligand was determined via HPLC.

| Ligand | *K*ᵢ (nM) TSPO | *K*ᵢ (nM) CRB | Log D |
|---|---|---|---|
| DPA-714 | 7.0 | > 10,000 | 2.44 |
| DPA-713 | 4.7 | > 10,000 | 2.44 |
| PK 11195 | 9.3 | > 10,000 | 3.35 |

### EXAMPLE 3 - Stimulation of neurosteroid production

In this example, DPA-714 was assessed for its ability to increase pregnenolone synthesis using a well developed steroidogenic assay (Selleri S, Bruni F, Costagli C, et al. 2-Arylpyrazolo[1,5-a]pyrimidin-3-yl acetamides. New potent and selective peripheral benzodiazepine receptor ligands. Bioorganic and Medicinal Chemistry. 2001;9:2661-2671).

The results of the pregnenolone assay (shown in Figure 1) demonstrate that DPA-714 stimulates steroid production with a notably greater potency compared to DPA-713 and the widely used PBR ligands, PK11195 and Ro5-4864.

### Cell Culture

Rat glioma C6 cells were cultured in Dulbecco's modified Eagle's medium supplemented with 10% FBS, 2 mM L-glutamine, 100 units/mL penicillin and 100 µg/mL streptomycin. Cultures were maintained in a humidified atmosphere of 5% CO₂/95% air at 37 °C.

### Steroid Biosynthesis Assay

C6 cells were seeded in 24-well plates at a density of ~1 x 10⁶ cells/well in a final volume of 1 ml. Prior to measurement of pregnenolone production the cells were washed three times with a simple salts medium consisting of 140 mM NaCl, 5 mM KCI, 1.8 mM CaCl₂, 1 mM MgSO₄, 10 mM glucose, 10 mM HEPES/NaOH, pH 7.4, plus 0.1% BSA. During experiments, cells were incubated with this simple salts medium in an air incubator at 37°C. In order to measure pregnenolone secreted into the medium, its further metabolism was blocked by the addition of trilostane (25 µM) and SU 10603 (10 µM) (inhibitors of 3β-hydroxysteroid dehydrogenase and 17α-hydroxylase, respectively) to the simple salts medium, as previously described (Campiani B, Nacci V, Fiorini I, et al, Synthesis, Biological, Actwily and SARs of Pyrrolobenzoxazepine Derivatives, a New Class of Specific "Peripheral-Type" Benzodiazepine Receptor Ligands, Journal of Medicinal Chemistry 1996; 39:3435-3450). The addition of the novel compounds and of PK 11195, Ro 5-4864, or clonazepam to the C6 cells was made by the complete change of the simple salts medium to a medium containing the appropriate concentration (40 µM) of compound. The final concentration of ethanol was constant for all the wells within each experiment and did not exceed 0.5% (v/v), a concentration, which on its own had no effect on steroid production. At the end of the incubation period (2 h) the cell medium was retained and centrifuged at 1500g for 10 min. The amount of pregnenolone secreted into the medium was quantified by radio immunoassay (RIA), using an antibody obtained from ICN Biochemical Inc., CA, USA, under the conditions recommended by the supplier. Cell protein concentration was measured according to a previously described method (Lowry OH, Rosenbrough NJ, Farr AL, Randall RJ. Protein measurement with the folin phenol reagent. J Biol Chem. 1951; 193; 265-275).

The results are shown in Figure 1. As show in Figure 1, DPA-714 stimulated pregnenolone synthesis at levels of 80% above baseline, displaying significantly greater potency than PK 11195 and Ro 5-4864. DPA-713 showed no effect on steroidogenesis in the same assay.

### EXAMPLE 4 - Ex Vivo [¹⁸F]DPA-714 Rodent Studies

Adult male Wistar rats weighing 300-320 g (Janvier, Le Genest-St-Isle, France) were used for ex vivo experiments. All procedures were carried out in accordance with the European Community Council Directive 86/609/EEC for the care of laboratory animals. Animals were kept on a 12-hour light/dark cycle (temperature 22.4 ± 0.5°C; hygrometry 40.3 ± 7.2%) and water and food were freely available.

*Quinolonic acid (QA) lesions:* Rats were anesthetised with isoflurane (4%, 500 mL/min), placed on a stereotaxic apparatus (Stoelting, Phymep, Paris, France) and were maintained under isoflurane 2% (500 mL/min) during surgery. The skull was exposed and small holes were made with use of a dental drill. Animals were unilaterally injected in the right striatum at the following coordinates: A, 0.7; L, -3; P, -5.5 mm from bregma (Paxinos and Watson, 1986). A cannula (gauge 25, Hamilton, Massy, France) was inserted and a solution of QA (300 nmol in 2 µL phosphate buffer, pH 7.4) was infused at a flow rate of 0.5 µL/min. The springe was left in place for 4 minutes before being removed to avoid backflow of QA. The bone was filled in with wax and the scalp was then sutured.

*Biodistribution studies:* Ex vivo biodistribution studies were performed with [¹⁸F]DPA-714 six days after the unilateral striatal lesion with QA. Rats were injected via the penis vein with 20-37 MBq [¹⁸F]DPA-714 in a mixture of water and EtOH (85/15) or following a pre-injection of either PK 11195 (n = 5, 5mg/kg), DPA-714 (n = 2, 1 mg/kg) or DPA-713 (n = 2; 1 mg/kg) 15 min before the radioligand. The rats that were only injected with the radioligand ([¹⁸F]DPA-714) and no pre-injection were the control group (n = 6). Animals were sacrificed 60 min after injection of [¹⁸F]DPA-714. Samples of peripheral tissue, i.e. blood, muscle, bone, liver, heart, adrenal and several brain areas (cerebellum, right and left striatum, right and left frontal cortex, right and left hippocampus) were removed, weighed and their radioactivity measured. For peripheral tissue, results were expressed as the ratio (%ID/g tissue) / (%ID/g blood) ± SEM. For brain, results were expressed as the ratio (%ID/g cerebral region) / (%ID/g cerebellum) ± SEM. Student t test was performed when the number of data was ≥ 5. Statistic significance was considered for p<0.05.

### Results

*Peripheral biodistribution:* Figure 2 shows the peripheral distribution of [¹⁸F]DPA-714 in each of the four QA lesioned rat groups; control (injected with radioligand only) and three pre-treatment groups (PK 11195, DPA-713 and DPA-714). Results from the control group show that the highest accumulation of [¹⁸F]DPA-714 was in the adrenals (71.62±35.06) and heart (59.26±29.06), whereas there was only minimal accumulation in the bone (6.30±0.97), liver (3.36±0.16) and muscle (2.11±1.13). The rats pre-treated with PK 11195 (5 mg/kg) demonstrated significant inhibition of radioligand uptake in the heart, bone and liver but not in the adrenals and muscle. In contrast, pre-treatment with either DPA-714 (1 mg/kg) or DPA-713 (1 mg/kg) was capable of blocking [¹⁸F]DPA-714 accumulation in all peripheral tissues.

*Cerebral biodistribution:* Figure 3 shows the cerebral distribution of [¹⁸F]DPA-714 in each of the four QA lesioned rat groups. Results from the control group, demonstrate a statistically significant increase in [¹⁸F]DPA-714 uptake in the right (lesioned) side of the striatum (4.81±0.47 vs 0.61±0.14; p<0.05) and frontal cortex (1.92 ±0.86 vs 0.68±0.13, p<0.05) compared to their corresponding non-lesioned counterpart. This was not observed in the hippocampus (0.74±0.13 vs 0.77±0.29). In the right striatum and frontal cortex, accumulation of [¹⁸F]DPA-714 was significantly decreased by pre-injection of PK 11195 (1.90±0.59 and 1.18±0.16, respectively, p<0.05). A visible decrease was also seen in these same regions following pre-injection of either DPA-714 (2.00±0.23 and 1.28±0.17, respectively) or DPA-713 (1.08±0.08 and 1.06±0.05, respectively). Interestingly, there was an increase of [¹⁸F]DPA-714 accumulation in the PK 11195 pre-treatment group compared to control, in the left striatum (1.25±0.48), left frontal cortex (0.99±0.04), right hippocampus (1.19±0.14) and left hippocampus (1.09±0.10). A similar trend was observed after pre-injection of DPA-714 as well as DPA-713.

### EXAMPLE 5 - In Vivo [¹⁸F]DPA-714 Baboon PET Studies

A normal male *Papio hamadryas* baboon aged 13 and weighing 23.1 kg was selected for PET scanning. The baboon was maintained and handled in accordance with the National Health and Medical Research Council (NHMRC) code of practice for the care and use of non-human primates for scientific purposes. The project application was approved by the Sydney South West Area Health Service (SSWAHS) Animal Ethics Committee. The radioligand doses injected were 100 MBq for [¹⁸F]DPA-714.

All PET data was acquired using a Siemens Biograph LSO PET-CT scanner in the Department of PET and Nuclear Medicine at Royal Prince Alfred Hospital, Australia. This dual modality device has a fully-3D PET scanner with 24 crystal rings and a dual slice CT scanner in the same gantry. It yields a reconstructed PET spatial resolution of 6.3 mm full width at half maximum (FWHM) at the centre of the field of view. The baboon was initially anaesthetized with ketamine (8 mg/kg, *im).* Anaesthesia was maintained with the use of an iv infusion of ketamine (Parnell Laboratory, Australia) in saline at a dose rate of 0.2 mg ketamine/kg/min. The baboon also received MgSO₄ (2 mL iv) given over half an hour plus atropine (1 mg *im)* plus maxalon (5 mg *im).* The head of the baboon was immobilised with plastic tape to minimise motion artifacts. A CT scan of the head was completed prior to radioligand injection. Acquisition of the PET data in list mode was commenced just prior to radioligand injection and continued for a period of 60 min. The blocking study involved pretreatment with PK 11195 (1.5 mg/kg) 5 minutes prior to radioligand injection whereas in the displacement study, cold DPA-714 (1 mg/kg) was administered at 20 minutes post injection of [¹⁸F]DPA-714. At the conclusion of each study the list mode data were sorted into a dynamic scan comprising 54 frames (20 x 30 s, 30 x 60 s and 4 x 300 s). The dynamic 3-D PET sinograms were rebinned using Fourier Rebinning (FORE) and reconstructed with filtered backprojection and CT data-based corrections for photon attenuation and scatter into 47 transaxial slices, each comprising 128 x 128 voxels. Reconstructed voxel dimensions were 0.206 x 0.206 x 0.337 cm. The radioligand uptake was converted to units of percent injected dose per volume of brain tissue (% dose/ mL) and plotted against time. An automated 3D registration algorithm was used to co-register the two reconstructed scans prior to ROI definition (Eberl S, Kanno I, Fulton RR, Ryan A, Hutton BF, Fulham MJ. 1996. Automated interstudy image registration technique for SPECT and PET. J Nucl Med 37(1):137-145).

Decay corrected time activity curves representing the variation in ligand concentration vs. time were constructed from selected slices for regions of interest over the thalamus, cerebellum, striatum, cortex, skull and whole brain. After the list-mode acquisition, a whole body PET-CT scan was performed, 2 minutes at each of the six bed positions, to determine other sites of radioligand uptake.

### Results

A baseline study was performed in which dynamic PET brain imaging commenced a few minutes preceding i.v. administration of [¹⁸F]DPA-714 (100 MBq in 2 ml saline, specific activity 270 GBq/µmol) and was terminated 60 mins post injection. This was followed by whole body acquisition for 2 minutes at each of the six bed positions. This PET summation images of transaxial brain slices obtained in the baseline study demonstrated that [¹⁸F]DPA-714 is capable of penetrating the blood brain barrier with considerable accumulation in the brain. No uptake was observed in the baboon skull.

To determine the specificity of [¹⁸F]DPA-714 binding, a blocking study was performed, involving pretreatment with TSPO ligand PK1195 (1.5 mg/kg). The PET summation images of transaxial brain slices obtained in this study demonstrated that PK11195 effectively blocked the uptake of [¹⁸F]DPA-714 in the brain. Lastly, a displacement study, in which cold DPA-714 (1 mg/kg) was administered 20 minutes post [¹⁸F]DPA-714 injection, was conducted to assess reversibility of radioligand binding.

Figure 4 shows the time activity curves (TAC) representing the uptake of [¹⁸F]DPA-714 in the whole baboon brain in each of the three PET studies (the baseline study, the blocking study and the displacement study). In the baseline study, [¹⁸F]DPA-714 reached a maximum uptake within ten minutes and remained at approximately the same level of uptake for the following 50 minutes. The displacement study appeared analogous to the baseline study during the first 20 minutes, however, after the injection of cold DPA-714, the curve rose into a sharp peak followed by complete washout of the radioligand. In contrast, pre-treatment with the TSPO specific ligand PK 11195, resulted in an initial increase in [¹⁸F]DPA-714 uptake, followed by rapid decline to washout level, identical to that seen in the displacement study.

Whole body images were also acquired directly following brain imaging to investigate other sites of radioligand uptake. These demonstrated the high uptake of [¹⁸F]DPA-714 in the heart, adrenals and salivary glands. Injection of cold DPA-714 at 20 minutes after radioligand administration led to complete displacement of [¹⁸F]DPA-714 binding in these peripheral regions (data not shown).

### EXAMPLE 6 - [¹⁸F]DPA-714 as a PET tracer for TSPO: A comparison with [11C]PK11195 in a rat model of HSV encephalitis

Many neurological diseases, including Parkinson's disease and herpes simplex encephalitis (HSE), are associated with neuroinflammation. Expression of the peripheral benzodiazepine receptor (PBR) is increased during neuroinflammation and can be visualised by positron emission tomography (PET) with [¹¹C]PK11195. However, [¹¹C]PK11195 shows low brain uptake and high non-specific binding and may not be sensitive enough to visualise mild inflammation. In this study, [¹⁸F]DPA-714 was evaluated in a rat model of HSE and compared to [¹¹C]PK11195 in the same model.

**Experimental:** [¹⁸F]DPA-714 was prepared by reacting of the corresponding tosylate precursor with K¹⁸F / kryptofix. The stability of [¹⁸F]DPA-714 was tested by TLC. Male Wistar rats were intranasally inoculated with the herpes simplex virus type-1 (10⁷ PFU in 1001 PBS) or PBS (control). Within a week following inoculation, replicating virus migrated into the brain and induced neuroinflammation. At day 6 or 7 after inoculation the rats received an i.v. injection of [¹⁸F]DPA-714 (559 MBq) or [¹¹C]PK11195 (7822 MBq) and dynamic PET scans (MicroPET Focus 220) were performed for 2 h and 1 h respectively, followed by ex vivo biodistribution.

**Results and Discussion:** [¹⁸F]DPA-714 was obtained in 20±5% radiochemical yield, with a specific activity of 10428 MBq/nmol and a radiochemical purity >99%. In-vivo, [¹⁸F]DPA-714 was slowly converted into more polar metabolites, with 78±1% of the radioactivity in rat plasma consisting of the parent compound at 2 h post tracer injection. The PET images of [¹⁸F]DPA-714 showed a low tracer uptake in control rats (n=3), which was significantly lower than [¹¹C]PK11195 uptake (n=5) at 1 h (p=0.01), and a slow tracer clearance from the brain (T_{1/2}>100 min). [¹⁸F]DPA-714 uptake in HSE rats was increased (90-150%) in olfactory and retrograde brain areas where HSV-1 accumulates. In these areas, [¹¹C]PK11195 uptake was not significantly increased.

**Conclusion:** These results demonstrate that [¹⁸F]DPA-714 is a useful tracer to visualise neuroinflammation, and is more sensitive than [¹¹C]PK11195 because of a better contrast between inflamed and noninflamed areas.

### EXAMPLE 7 - Toxicity study for DPA-714

In this study, the possible toxicity of DPA-714 was evaluated after a single administration by the intravenous route in the rat.

The study involved 2 groups of 20 SPF Sprague-Dawley rats (each of 5 males and 5 females), 7 weeks old and weighing between 193.1 g and 215 g for males and between 160.6 g and 180.1 g for females on the day of randomisation. Animals were purchased from Charles River Laboratories France (Domaine des Oncins - 69592 L'Arbresle Cedex, France).

Groups were as follows:
- Group 1: dosed with the vehicle (*i.e*. 0.9%NaCl / ethanol (9/1, (v/v)).
- Group 2: dosed with DPA-714 at 5 mL/kg

The test item DPA-714 diluted at 1/10 in its vehicle (*i.e*. 0.9% NaCl / ethanol 9/1, (v/v)) or the vehicle were administered after filtration with 0.2 µm filter to animals by intravenous route as a bolus over about 30 seconds in a volume of 5 mL/kg.

Animals were weighed on the day of randomisation, on D7, D14 and D15 (day of necropsy).

General observations were performed on D1, 60 minutes ±30 minutes after dosing, again between 3 and 4 hours post-dose and then once a day for 14 days. Functional and neurobehavioural tests were also assessed on D1, 60 minutes ± 30 minutes after dosing and then on D7 and D14. Mortality was recorded twice a day for 14 days. All animals surviving at the end of the 14-day period were submitted to gross necropsy.

Under the experimental conditions adopted, animals dosed with the vehicle (*i.e.* 0.9% NaCl / ethanol 9/1, v/v)) did not exhibit any significant sign and the body weight gain was normal. No abnormality attributed to the vehicle was noticed in organs examined at the necropsy.

DPA-714 diluted at 1/10 in the vehicle (*i.e.* final concentration of 0.05 mg/mL) and administered by the intravenous route at 5 mL/kg induced no mortality. No effect on body weight gain of males and females was seen when compared with the control group. No relevant clinical sign was observed in animals which were given DPA-714 diluted at 1/10 in the vehicle by the intravenous route at 5 mL/kg. No gross lesion was observed at necropsy.

Under the experimental conditions adopted, DPA-714 (batch No. 140306) administered once by the intravenous route in the Sprague-Dawley rat at 5 mL/kg did not induce any toxicity sign.

Therefore, the LD₅₀ of DPA-714 (batch No. 140306) is higher than 5 mL/kg in male and female Sprague-Dawley rat when administered by the intravenous route.

### Comments

The *in vitro* binding studies using rat kidney membranes and [³H]PK 11195 (Example 2) demonstrated that DPA-714 specifically binds the TSPO with high affinity. Biodistribution studies using microPET in both normal and lesioned rats (Example 4) showed that [¹⁸F]DPA-714 is a highly sensitive and accurate radioligand for detecting regions of altered TSPO expression. These rat studies also proved that [¹⁸F]DPA-714 has favourable kinetics and stability *in vivo.*

PET imaging in normal baboon brain (Example 5) reaffirmed the results observed in the rat studies. The results of Example 5 show [¹⁸F]DPA-714 binds specifically and selectively to the TSPO and shows no species dependence in TSPO binding.

A novel feature of DPA-714 is its marked functional activity in the pregnenolone steroidogenic assay (Example 3). In fact, DPA-714 is twice more active at stimulating neurosteroid synthesis than PK 11195, the most widely used TSPO ligand, in the same assay.

In addition, DPA-714 has a higher binding affinity (*K*ᵢ= 7.0 nM) compared to PK 11195 (*K*ᵢ=9.3 nM) (Example 2). This higher binding affinity, together with the superior *in vivo* kinetics and lower lipophilicity of DPA-714, means that [¹⁸F]DPA-714 is better able to label the TSPO.

[¹⁸F]DPA-714 can, therefore, be used as an accurate *in vivo* marker of microglial activation. More specifically, [¹⁸F]DPA-714 can be used together with PET as a means of detecting initial neuropathological events in a number of neurodegenerative disorders. This radioligand can also be used as a tool for studying disease progression and treatment effectiveness. The types of diseases for which [¹⁸F]DPA-714 could be used as a tool for studying disease progression and treatment effectiveness include Alzheimer's disease (AD), Parkinson's disease (PD), Huntington's disease, multiple sclerosis (MS), multiple system atrophy (MSA), epilepsy, encephalopathy, stroke and brain tumours. The high specific binding and high selectivity of [¹⁸F]DPA-714 along side its favourable *in vivo* kinetics make it suitable for this application.

## Claims

1. A compound of formula (I) 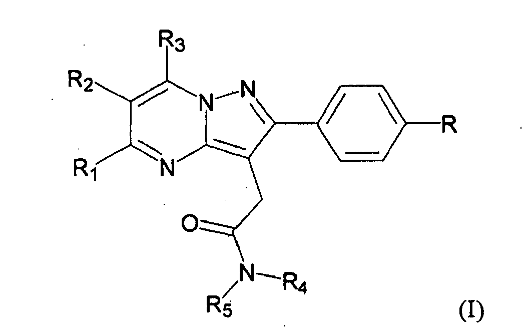 wherein:
R is H, halo, alkyl optionally substituted with halo, or alkoxy optionally substituted with halo;
R₁, R₂ and R₃ are each independently H or a hydrophobic group wherein the hydrophobic group is independently selected from C₁₋₆alkyl optionally substituted with halo, aryl optionally substituted with halo, NHC₁₋₆alkyl optionally substituted with halo, OC₁₋₆alkyl optionally substituted with halo, SC₁₋₆alkyl optionally substituted with halo, COOR₆ where R₆ is C₁₋₆alkyl optionally substituted with halo, (CH-₂)ₙOR₆ where R₆ is C₁₋₆alkyl optionally substituted with halo, and n is an integer, and polyethers optionally substituted with halo; and
R₄ and R₅ are each independently alkyl optionally substituted with halo, or alkoxy optionally substituted with halo,
radiolabeled with a radioisotope selected from ¹⁸F, ¹²³I, ⁷⁶Br, ¹²⁴I and ⁷⁵Br,
or a salt thereof.

2. The compound according to claim 1, or a salt thereof, wherein R is C₁₋₆alkyl optionally substituted with halo or C₁₋₆alkoxy optionally substituted with halo.

3. The compound according to claim 1 or claim 2, or a salt thereof, wherein R₄ and R₅ are each independently selected from C₁₋₆alkyl optionally substituted with halo and C₁₋₆alkoxy optionally substituted with halo.

4. A pharmaceutical composition comprising a compound according to any one of claims 1 to 3, or a pharmaceutically acceptable salt thereof, and a pharmaceutically acceptable carrier.

5. A compound according to any one of claims 1 to 3, or a pharmaceutically acceptable salt thereof, for use in imaging translocator protein (18 kDa) in a subject.

6. A compound according to any one of claims 1 to 3, or a pharmaceutically acceptable salt thereof, for use in diagnosing a neurodegenerative disorder in a subject.

## Patentansprüche

1. Eine Verbindung der Formel (1) 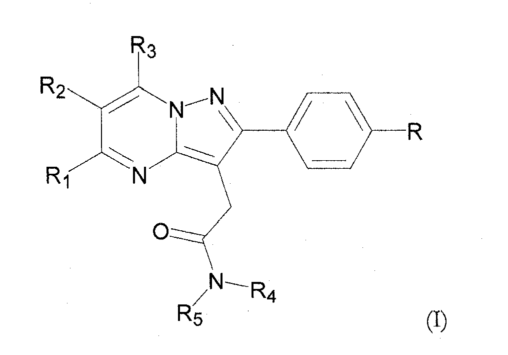 wobei:
R ein H, Halo, Alkyl optional substituiert mit Halo oder Alkoxy optional substituiert mit Halo ist;
R₁, R₂, und R₃ jeweils unabhängig H oder eine hydrophobe Gruppe sind, wobei die hydrophobe Gruppe unabhängig ausgewählt ist aus C₁₋₆-Alkyl optional substituiert mit Halo, Aryl optional substituiert mit Halo, NHC₁₋₆-Alkyl optional substituiert mit Halo, OC₁₋₆-Alkyl optional substituiert mit Halo, SC₁₋₆-Alkyl optional substituiert mit Halo, COOR₆ wobei R₆ C₁₋₆-Alkyl optional substituiert mit Halo ist, (CH₂)ₙOR₆ wobei R₆ C₁₋₆-Alkyl optional substituiert mit Halo ist und n ganzzahlig ist, und Polyethern optional substituiert mit Halo; und
R₄ und R₅ jeweils unabhängig Alkyl optional substituiert mit Halo oder Alkoxy optional substituiert mit Halo sind,
radioaktiv-markiert mit einem Radioisotop ausgewählt aus ¹⁸F, ¹²³I, ⁷⁶Br, ¹²⁴I und ⁷⁵Br,
oder ein Salz davon.

2. Die Verbindung entsprechend Anspruch 1 oder einem Salz davon, wobei R ein C₁₋₆-Alkyl optional substituiert mit Halo oder C₁₋₆-Alkoxy optional substituiert mit Halo ist.

3. Die Verbindung entsprechend Anspruch 1 oder Anspruch 2, oder einem Salz davon, wobei R₄ und R₅ jeweils unabhängig ausgewählt aus C₁₋₆-Alkyl optional substituiert mit Halo und C₁₋₆-Alkoxy optional substituiert mit Halo sind.

4. Ein pharmazeutische Zusammensetzung umfassend einer Verbindung entsprechend irgendeinem der Ansprüche 1 bis 3, oder einem pharmazeutisch verträglichen Salz davon, und einem pharmazeutisch verträglichen Träger.

5. Eine Verbindung entsprechend irgendeinem der Ansprüche 1 bis 3, oder einem pharmazeutisch verträglichen Salz davon, zur Verwendung in der Bildgebung des Translokator-Proteins (18 kDa) in einem Subjekt.

6. Eine Verbindung entsprechend irgendeinem der Ansprüche 1 bis 3, oder einem pharmazeutisch verträglichen Salz davon, zur Verwendung in der Diagnose einer neurodegenerativen Störung in einem Subjekt.

## Revendications

1. Composé de formule (I) 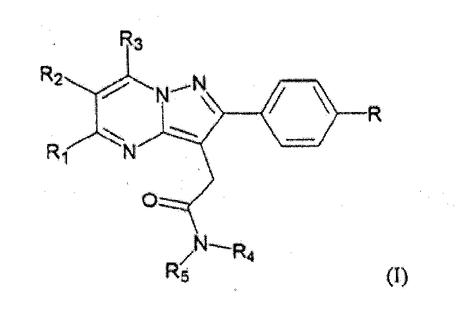 dans laquelle :
R est H, un halo, un alkyle éventuellement substitué avec un halo ou un alcoxy éventuellement substitué avec un halo ;
R₁, R₂ et R₃ sont chacun indépendamment H ou un groupe hydrophobe, dans lequel le groupe hydrophobe est choisi indépendamment parmi un alkyle en C₁ à C₆ éventuellement substitué avec un halo, un aryle éventuellement substitué avec un halo, un NHalkyle en C₁ à C₆ éventuellement substitué avec un halo, un O-alkyle en C₁ à C₆ éventuellement substitué avec un halo, un S-alkyle en C₁ à C₆ éventuellement substitué avec un halo, COOR₆ où R₆ est un alkyle en C₁ à C₆ éventuellement substitué avec un halo, (CH₂)ₙOR₆ où R₆ est un alkyle en C₁ à C₆ éventuellement substitué avec un halo et n est un nombre entier, et des polyéthers éventuellement substitués avec un halo ; et
R₄ et R₅ sont chacun indépendamment un alkyle éventuellement substitué avec un halo ou un alcoxy éventuellement substitué avec un halo,
radiomarqué avec un radio-isotope choisi parmi ¹⁸F, ¹²³I, ⁷⁶Br, ¹²⁴I et ⁷⁵Br,
ou un sel de celui-ci.

2. Composé selon la revendication 1 ou sel de celui-ci, dans lequel R est un alkyle en C₁ à C₆ éventuellement substitué avec un halo ou un alcoxy en C₁ à C₆ éventuellement substitué avec un halo.

3. Composé selon la revendication 1 ou la revendication 2 ou sel de celui-ci, dans lequel R₄ et R₅ sont choisis chacun indépendamment parmi un alkyle en C₁ à C₆ éventuellement substitué avec un halo et un alcoxy en C₁ à C₆ éventuellement substitué avec un halo.

4. Composition pharmaceutique comprenant un composé selon l'une quelconque des revendications 1 à 3 ou un sel pharmaceutiquement acceptable de celui-ci, et un support pharmaceutiquement acceptable.

5. Composé selon l'une quelconque des revendications 1 à 3 ou sel pharmaceutiquement acceptable de celui-ci, pour une utilisation dans l'imagerie d'une protéine de translocation (18 kDa) chez un sujet.

6. Composé selon l'une quelconque des revendications 1 à 3 ou sel pharmaceutiquement acceptable de celui-ci, pour une utilisation dans le diagnostic d'un trouble neurodégénératif chez un sujet.
